# EUROPEAN PATENT SPECIFICATION

(11) **EP 2 125 064 B1**
(45) Date of publication and mention of the grant of the patent: **26.04.2017**
(21) Application number: 08728017.8
(22) Date of filing: 22.01.2008
(51) Int. Cl.: A61L 31/02, A61L 31/08, A61L 31/14

(54) **IMPLANTABLE MEDICAL ENDOPROSTHESES**
IMPLANTIERBARE MEDIZINISCHE ENDOPROTHESEN
ENDOPROTHÈSES MÉDICALES IMPLANTABLES

(30) Priority: 26.01.2007 US 886705 P
(43) Date of publication of application: 02.12.2009
(73) Proprietor: Boston Scientific Limited, Hamilton HM11 (BM)
(72) Inventor: ATANASOSKA, Liliana, Edina, MN 55436 (US); WEBER, Jan, NL-6228 GJ Maastricht (NL); SCHEWE, Scott R., Eden Prairie, MN 55346-1334 (US); WARNER, Robert W., Woodbury, MN 55125 (US)
(74) Representative: Peterreins Schley
(86) International application number: PCT/US2008/051607
(87) International publication number: WO 2008/091835

(56) References cited:
- WO-A-2006/024125
- WO-A-2006/082221
- DE-A1- 10 357 281
- US-A- 4 818 572
- FORTIMEDIX BV: 'Kaon Bare Coronary stent', [Online] company brochure, 2 pages Retrieved from the Internet: <URL:http://www.fortimedix.com/upload/File/ pdf/kaon-bare.pdf> [retrieved on 2013-08-16]

## Description

### TECHNICAL FIELD

This disclosure generally relates to implantable medical endoprostheses, as well as related systems and methods.

### BACKGROUND

Implantable medical endoprostheses can be placed within body lumens. Examples of implantable medical endoprostheses include stents, such as balloon-expandable stents and self-expanding stents.

WO 2006/082221 A1 is directed to a process for manufacturing a drug delivery material, the process comprising the steps of encapsulating at least one biologically and/or therapeutically active agent in a shell; combining the encapsulated active agent with a sol; and converting the resulting combination into a solid or semi-solid drug delivery material. The invention further comprises drug delivery materials prodicible by such a process, as well as medical implants comprising such drug delivery material.

WO 2006/024125 A1 discloses a method of modifying the surface of a metal substrate to improve the surface coverage of a coating applied to the substrate. The method comprises heating at least the surface of the substrate to a temperature within the range of approximately 175 - 400 °C; and applying at least one layer of the coating to the substrate.

DE 10357281 A1 discloses a stent which is at least partially provided with a degradation-inhibiting, biocompatible coating. The coating comprises magnesium fluoride as a main component, and may have different thickness or structural form in different areas of the stent.

US-A4 818 572 discloses a process for production of a calcium phosphate compound-coated composite material, comprising oxidizing a metallic substrate to form a layer of the oxide of the metal component of the metallic substrate on the surface thereof, and forming a coating layer of a calcium phosphate compound on the surface of the oxide layer.

### SUMMARY

The invention relates to an implantable medical endoprosthesis as further defined in appended claim 1. The invention further relates to a delivery system for said implantable medical endoprosthesis as further defined in appended claim 12. Embodiments of the invention can include one or more of the following advantages.

Anodizing portions of a stent body to produce an oxide layer adjacent to a surface of the body can permit a sol-gel coating to be deposited on the surface of the body. Some sol-gel coatings applied directly to a surface of certain stents such as stents formed from magnesium- containing materials will react chemically with the stent body, leading to pre-implantation erosion of the stent body. However, the applied sol-gel coatings generally do not react with the oxide layer produced by anodizing a portion of the stent body. One or more sol-gel coatings can therefore be applied to a surface of the stent body without resulting in erosion of the stent prior to implantation.

The composition of sol-gel coatings can be varied to control an average erosion time of a stent within a body lumen. For example, by applying to the stent body a sol-gel coating with a short erosion time in body lumens, exposure of the coated portions of the stent body to the lumen environment following erosion of the sol-gel coating occurs relatively quickly, and the average erosion time of the stent in a body lumen is relatively short. By applying a sol-gel coating with a long erosion time in body lumens, exposure of the coated portions of the stent body to the lumen environment following erosion of the sol-gel coating occurs relatively slowly, and the average erosion time of the stent in a body lumen is relatively long. Thus, by choosing the composition of sol-gel coatings applied to the stent body, an average erosion time of the stent in a body lumen can be varied.

One or more encapsulated agents can be contained within one or more sol-gel coatings for controlled delivery of the agents to sites within body lumens. For example, sol-gel coatings can include encapsulated pH buffers, enzymes, antibodies, proteins, chelating agents, and other chemical species. These encapsulated agents are released as erosion of the sol-gel coating occurs within a body lumen. By selecting a suitable composition of a sol-gel coating (and thereby determining a particular erosion rate of the coating within a body lumen), an approximate release time for encapsulated agents therein can be selected.

Multiple sol-gel coatings can be used to release different encapsulated agents at different times. For example, an outermost sol-gel coating in a coating stack, which is directly exposed to the lumen environment upon implantation of the stent, will erode prior to inner sol-gel coatings positioned closer to the stent body, and which are exposed to the lumen environment only after the outermost sol-gel coating has eroded.

By including encapsulated agents in different sol-gel coatings, different average release times for the encapsulated agents can be selected as sequential erosion of each of the sol-gel coatings occurs in a body lumen.

Sol-gel coatings can be applied in patterns to surfaces of stents to control the shapes of stent fragments produced during erosion within a body lumen. For example, sol-gel coatings can be applied in patterns such as rings, dots, hatched patterns, and other patterns to surfaces of a stent body to increase the average erosion time of the coated portions of the stent body in body lumens. Sol-gel coatings that differ in either or both of composition and thickness can be applied to different portions of the stent body to produce coated regions of the stent that have different average erosion times according to the properties of one or more sol-gel layers applied thereto.

Other features and advantages of the invention will be apparent from the description, drawings, and claims.

### DESCRIPTION OF DRAWINGS

FIG. 1A is a side view of an embodiment of a stent having a sol-gel coating.
FIG. 1B is a cross-sectional view of the stent shown in FIG. 1A.
FIG. 2A is a side view of an embodiment of a stent having multiple concentric sol-gel coatings.
FIG. 2B is a cross-sectional view of the stent shown in FIG. 2A.
FIG. 3A is a side view of an embodiment of a stent having sol-gel coatings on multiple stent surfaces. ' FIG. 3B is a cross-sectional view of the stent shown in FIG. 3A.
FIG. 4A is a side view of an embodiment of a stent having multiple non-concentric sol- gel coatings.
FIG. 4B is a cross-sectional view of the stent shown in FIG. 4A.
FIG. 5A is a side view of an embodiment of a stent having a sol-gel coating applied to selected portions of the stent.
FIG. 5B is a cross-sectional view of the stent shown in FIG. 5A.
FIG. 6 is a plan view of an embodiment of a stent having a sol-gel coating formed in a pattern of dot-like shapes.
FIG. 7 is a plan view of an embodiment of a stent having a sol-gel coating formed in a crosshatched pattern
FIG. 8 is a perspective view of an embodiment of a stent having a sol-gel coating and a plurality of holes extending through the stent body.
FIG. 9 is a perspective view of an embodiment of a coil stent with a sol-gel coating.
FIG. 10 is a perspective view of another embodiment of a coil stent with a sol-gel coating.
FIG. 11 is a perspective view of a further embodiment of a stent with a sol-gel coating.
FIG. 12 is a perspective view of an embodiment of a woven stent with a sol-gel coating.
FIGS. 13-15 arc side views of an embodiment of an endoprosthesis delivery system during use.

Like reference symbols in the various drawings indicate like elements.

### DETAILED DESCRIPTION

This disclosure relates to implantable medical endoprostheses such as stents (e.g., balloon-expandable stents, self-expanding stents) that have sol-gel coatings. The sol-gel coatings can be used, for example, to control erosion times of the implantable medical endoprostheses and to deliver agents to selected body sites at selected times.

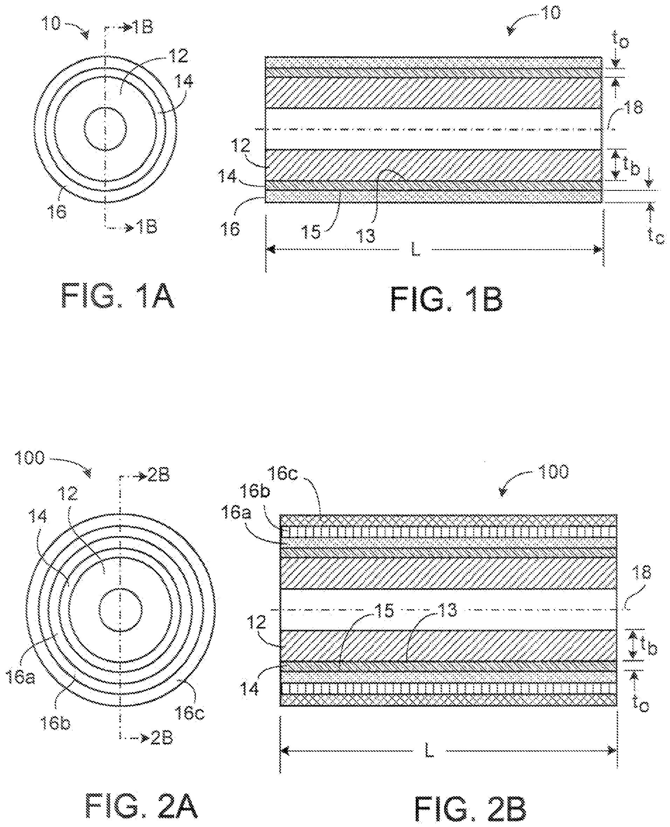

FIGS. 1A and IB show side and cross-sectional views, respectively, of a stent 10. The cross-sectional view shown in FIG. 1B is taken along the 1 B-1 B section line shown in FIG. 1A. Stent 10 has a tubular stent body 12 that has a length L measured in a direction parallel to longitudinal axis 18, and a thickness t_{b} measured in a radial direction perpendicular to axis 18. An intermediate layer 14 is disposed on stent body 12 and has a thickness to measured in a radial direction perpendicular to axis IS. A sol-gel coating 16 is disposed on intermediate layer 14. Sol-gel coating 16 has a thickness measured in a radial direction perpendicular to axis 18. As referred to herein, a sol-gel material is a material that undergoes a transition from a suspension of solid particles in a liquid (sol) to a gel.

In general, stent body 12 is formed of a magnesium-containing material. For example, in certain embodiments, stent body 12 can be formed from magnesium. In certain other embodiments, stent body 12 can be formed from magnesium-containing alloy materials. In general, many different magnesium-containing materials can be used to form stent body 12. For example, stent body 12 can be formed from magnesium alloy materials such as AZ91, AZ91D, AZ91E, AZ92, ZE41. QE22, WE43A, and EZ33. Stent body 12 can be formed from alloys that include magnesium and one or

more of the following materials: aluminum, cerium, copper, lanthanum, lithium, manganese, neodymium, silver, thorium, yttrium, zinc, and zirconium.

The length L of stent body 12 can generally be selected as desired. In some embodiments, L can be chosen according to a particular intended use for stent 10, e.g., according to physiological properties of a body lumen where stent 10 will be implanted. In certain embodiments, L can be about 5 mm or larger (e.g., about 10 mm or larger, about 20 mm or larger, about 30 mm or larger, about 40 mm or larger). In some embodiments, L can be about 250 mm or smaller (e.g., about 200 mm or smaller, about 150 mm or smaller, about 100 mm or smaller).

The thickness t_{b} of stent body 12 can generally be selected as desired. In some embodiments, t_{b} can be about 0.05 mm or more (e.g., about 0.1 mm or more, about 0.2 mm or more, about 0.5 mm or more, about 1 mm or more). In certain embodiments, t_{b} can be about 5 mm or less (e.g., about 4 mm or less, about 3 mm or less, about 2 mm or less).

In general, intermediate layer 14 is formed as a material that can act as a bander between the environment of a body lumen and the material of stent body 12. Thus, while stent body 12 is typically formed of a material that erodes when exposed to the environment in a body lumen, intermediate layer 14 can increase the average erosion time of stent 10 within a body lumen by initially preventing contact between the material of stent body 12 and the body lumen environment. Optionally, intermediate layer 14 can be formed of a material that erodes over time when exposed to the environment in a body lumen, thereby exposing the material of stent body 12 to the environment of the body lumen, leading to erosion of stent body 12.

Additionally or alternatively, intermediate layer 14 can provide an adhesion layer for sol- gel coating 16. In general, certain precursor materials used to form sol-gel coaling 16 can react with the material from which stent body 12 is formed, which can result in undesirable erosion of stent body 12 prior to use of stent 10. Intermediate layer 14 can reduce (e.g., prevent) such undesirable erosion because layer 14 can be formed of a material that is relatively inert to the precursor materials of coating 16 and/or coating 16 itself. Therefore, by first forming intermediate layer 14 supported by stent body 12, sol-gel coating 16 can be included as a layer supported by stent body 12, even though stent body 12 may be formed of a material (e.g., a magnesium material) that ordinarily may undergo erosion when contacted with certain precursor materials that can be used to form sol gel layer 16.

Intermediate layer 14 generally includes oxides of the material of stent body 12 (e.g., magnesium oxides). For example, layer 14 can be formed by oxidizing one or more portions of the material from which stent body 12 is formed so that layer 14 is integral with stent body 12. In some embodiments, this can involve an anodizing process. An example of such a process is as follows. An electrolytic oxidation process is used to anodize portions of stent body 12. For example, stent body 12 is placed in an electrolyte solution along with two electrodes, and an electric potential is applied across the electrodes to cause current to flow in the electrolyte solution.

In general, the electrolyte solution can include any appropriate materials. For example, in some embodiments, the electrolyte solution can include one or more hydroxide materials (e.g., NaOH). In certain embodiments, the electrolyte solution can include one or more oxide materials (e.g., NaAlO₂). Further, the electrolyte solution can also include other materials such as magnesium and/or iron silicates, titanates, zirconates, niobates, tantalates, tungstenates, ruthenates, iridates, platinates, and ferrates.

In certain embodiments, voltages in a range from about 10 V to about 600 V (e.g., from about 50 V to about 500 V, from about 100 V to about 400 V, from about 150 V to about 300 V) can be used to anodize portions of stent body 12. Voltages applied across the electrodes can, in general, be constant in time or can be time-varying. For example, in some embodiments, a DC voltage can be applied across the electrodes in the electrolyte solutions. In certain embodiments, time-varying voltages (e.g., AC voltages, pulsed voltages) can be applied across the electrodes.

In some embodiments, a voltage can be applied across the electrodes for about ten seconds or more (e.g., about 30 seconds or more, about one minute or more, about 10 minutes or more, about 20 minutes or more, about 30 minutes or more, about 45 minutes or more, about 60 minutes or more). In certain embodiments, a voltage can be applied across the electrodes for about 12 hours or less (e.g., about 6 hours or less, about 3 hours or less, about 2 hours or less, about 1 hour or less).

An electrochemical reaction occurs in the electrolyte solution that converts magnesium in stent body 12 to magnesium oxide. The electrochemical reaction generally occurs on surfaces of stent body 12 that are exposed to the electrolyte solution. In certain embodiments, the surfaces of stent body 12 are all fully exposed to the electrolyte solution, so that oxide layers are formed on each of the exposed surfaces. In some embodiments, portions of the surfaces of stent body 12 arc masked (e.g., with a masking agent) to prevent exposure of the masked portions to the electrolyte solution. As a result, intermediate layer 14 is not formed in the masked portions of stent body 12.

The concentration of magnesium oxide in intermediate layer 14, measured along a radial direction perpendicular to axis 18, is generally not uniform. Instead, because forming magnesium oxide depends upon contact between the material of stent body 12 and the electrolyte solution, larger
concentrations of magnesium oxide are formed nearer an outer surface of 15 of layer 14 than nearer an inner surface 13 of layer 14. As a result, a gradient in magnesium oxide concentration is formed in intermediate layer 14, with the concentration of magnesium oxide generally increasing across layer 14 in the radial direction from inner surface 13 to outer surface 15.

The anodizing process can produce pores in intermediate layer 14. The distribution of pores produced in intermediate layer 14 follows a gradient that is similar to the gradient in concentration of magnesium oxide. That is, the concentration of pores in layer 14 generally increases in the radial direction from inner surface 13 to outer surface 15. In the region of outer surface 1 the concentration of pores can be relatively high.

In general, the adhesion strength of sol-gel coating 16 to intermediate layer 14 can be controlled by choosing a particular pore density in intermediate layer 14, particularly in the region of outer surface 15. Larger pore densities in the region of surface 15 can provide a larger effective surface area to which sol-gel coating 16 can adhere, which generally enhances the adhesion between layers 14 and 16.

The thickness t" of intermediate layer 14 can also be selected by adjusting a composition of the electrolyte solution and the voltage applied across the electrodes during the anodizing process. In some embodiments, the electrolyte solution and applied voltage can be adjusted so that t_{c}, is about 1 pm or more (c.g., about 2 *fim* or more, about 5 *pm* or more, about 10 *pm* or more, about 50 *pm* or more). In certain embodiments, f, can be about 5 mm or less (e.g., about 3 mm or less, about 1 mm or less, about 750 *pm* or less, about 500 *pm* or less, about 250 *pm* or less, about 150 *pm* or less, about 100 *pm* or less).

In general, sol-gel coating 16 can be prepared as desired. In some embodiments, sol-gel coating 16 is applied to intermediate layer 14. In some embodiments, the thickness L of sol-gel coating 16 can be about 1*µm* or more (e.g., about 2 *µm* or more, about 5 *µm* or more, about 10 *µm* or more, about 50 *µm* or more). In certain embodiments, tₒ can be about 5 mm or less (e.g., about 3 mm or less, about 1 mm or less, about 750 *µm* or less, about 500 *µm* or less, about 250 *µm* or less, about 150 *µm* or less, about 100 *µm* or less).

Sol-gel coating 16 can be formed from a variety of materials. In general, sol-gel coating 16 is formed from one or more bioerodible materials, e.g., materials which erode when exposed to the environment of a body lumen. In some embodiments, sol-gel coating 16 can include one or more bioerodible metal materials such as magnesium, zinc, iron, aluminum, titanium, iridium, tungsten, tantalum, niobium and zirconium. For example, sol-gel coating 16 can include magnesium oxide materials.

These materials can be derived from sol-gel precursor materials such as magnesium acetates, magnesium ethoxides, magnesium methoxides, and other magnesium alkoxides. As another example, sol-gel coating 16 can include mixed magnesium-zinc oxide materials according to the average formula MgₓZn].ₛO. These materials can be derived from sol-gel precursors such as magnesium acetates and zinc acetates. As yet another example, sol-gel coating 16 can include mixed oxide materials according to any one of the average formulas MgₓAl_{y}O, MgₓTi_{y}O, and MgₓZr_{y}O. These materials can be derived from sol- gel precursors that can include acetates and/or alkoxides of Mg, Al, Ti, and Zr. As a further example, sol-gel coating 16 can include composite materials such as alginate-magnesium aluminum silicate materials that can be formed into a nanocomposite film.

In certain embodiments, sol-gel coating 16 can include polymer materials such as polyvinyl alcohol (PVA), polyethylene glycol (PEG), and other polymer materials. In some embodiments, sol-gel coating 16 can be formed from hybrid materials that include both polymers and metals. For example, sol-gel coating 16 can be formed from a bioerodible material that includes MgO, CaO, SiCb, P₇O₅, and PVA. As another example, sol-gel coating 16 can be formed from a mixture of MgOₓ and TiOₓ combined with PEG to form a nanoporous coating layer.

Sol-gel coating 16 is formed by a sol-gel process applied to selected portions of stent 10. For example, in some embodiments, the sol-gel process is applied to deposit one or more sol-gel layers on portions of stent 10 that include intermediate layer 14. Sol-gel precursors are selected according to the desired composition of sol-gel layer 16. For example, to produce a sol-gel layer that includes magnesium, magnesium-based precursors are used. Suitable precursors for metal oxides include metal alkoxides and metal acetates. For example, precursors for magnesium include magnesium methoxides, magnesium elhoxides, magnesium isopropoxides. magnesium acetates, and other magnesium alkoxides. To produce a sol-gel coating 16 that includes more than one material (e.g., MgO and TiO), multiple different sol-gel precursors can be used.

Steps that can be performed to deposit a sol-gel coating 16 will be discussed subsequently with reference to a coating 16 formed from MgO. However, these steps can generally be performed to deposit coatings formed from any of the sol-gel materials disclosed above. First, the sol-gel precursor (e.g., a magnesium alkoxide) is dissolved in an alcohol such as methanol or ethanol.

Second, a catalyst material (e.g., formed from one or more acidic materials such as hydrochloric acid, acetic acid, nitric acid, and/or one or more basic materials) is used to catalyze hydrolysis of the precursor to form a hydroxide (e.g., magnesium hydroxide). Third, the hydroxide is dehydrated (e.g., by heal curing or another type of dehydration process) to yield an oxide (e.g., magnesium oxide) as a sol-gel coating 16.

Between the first and second steps, or between the second and third steps, the sol-gel precursor in solution is contacted to stent 10. Various methods can be used to perform the contacting. For example, in some embodiments, stent 10 can be coated with sol-gel precursor solution by dip coating. In certain embodiments, stent 10 can be coated using methods such as spray coating, brush coating, and/or spin coating. In some embodiments, stent 10 can be coated with sol-gel precursor solution using contact printing methods such as pin printing. Contact printing methods can be used to apply sol-gel precursor solution to stent 10 in complex patterns, for example.

In some embodiments, the composition and/or thickness t_{c} of sol-gel coating 16 are/is selected to control an average erosion time of stent 10 in body lumens. For example, in general, the larger the thickness t_{c} of sol-gel coating 16, the larger the average erosion time of sol-gel coating 16, and therefore the larger the average erosion time of stent 10. In contrast, the smaller the thickness **_{tc}** of sol-gel coating 16, the smaller the average erosion time of both sol-gel coating 16 and stent 10. As another example, to produce a stent 10 having a relatively long average erosion time in body lumens, sol-gel coating 16 can be formed from one or more materials that have a relatively low erosion rate. Examples of suitable materials with relatively low erosion rates include TiOₓ. TaOₓ, and WO₃. To produce a stent 10 having a relatively small average erosion time in body lumens, sol-gel coating 16 can be formed from one or more materials that have a relatively high erosion rate. Examples of suitable materials with relatively high erosion rates include materials such as MgOₓ. FeOₓ, and ZnOₓ.

In certain embodiments, sol-gel coating 16 can include one or more of various types of encapsulated agents. In such embodiments, the encapsulating agent(s) can be disposed in a matrix material, such as, for example, albumin, alginate, cellulose derivatives, collagen, fibrin, gelatin, polysaccharides, polyesters, polyethylene glycol, and polyanhydrides. Examples of encapsulating agents can include drugs, pH buffers, enzymes, antibodies, proteins, chelating agents, and other chemical species. Each of these different types of agents can be used to perform specific functions in a body lumen where stent 10 is implanted, or in other body sites. For example, one or more encapsulated pH buffers (e.g., an ion-exchange resin material) can be included in sol-gel coating 16 to maintain the pH within a particular range in a body lumen where stent 10 is implanted. Examples of suitable pH buffers include ion exchange resins such as pharmaceutical grade Amberite IRP-64 and 1RP-88. As stent 10 undergoes erosion in the lumen, the pH of the surrounding environment increases due to chemical reactions involving materials such as magnesium. The one or more encapsulated pH buffers within sol-gel coating 16 arc released as erosion occurs. The pH buffers counteract the increase in pH due to magnesium reactions, maintaining the pH in the environment of the lumen within a particular range,

As another example, one or more encapsulated chelating agents within sol-gel coating 16 can be used to control an average erosion time of stent 10 within a body lumen. For example, as stent 10 undergoes erosion, the encapsulated chelating agents (e.g., porphyrins) are released from sol-gel coating 16. The chelating agents chelate eroded magnesium, reducing a rate at which magnesium fragments detach from stent 10. By controlling the types and amounts of chelating agents within sol-gel coating 16, an erosion rate of stent 10 (and therefore, an average erosion time of stent 10) within body lumens can be controlled. Examples of suitable chelating agents include porphyrins, EDTA, Amberlite resins, and modified Pluronics.

As a further example, various types of encapsulated therapeutic agents such as drugs, enzymes, proteins, and antibodies can be included in sol-gel coating 16. As erosion of stent 10 occurs within a body lumen, the therapeutic agents are released to the environment surrounding the lumen, where they can perform specific treatment functions.

Therapeutic agents that can be included in sol-gel coating 16 include pharmaceutically active compounds, nucleic acids with and without carrier vectors such as lipids, compacting agents (such as histones), virus (such as adenovirus, adeno-associated virus, retrovirus, lentivirus and a-virus), polymers, antibiotics, hyaluronic acid, gene therapies, proteins, cells, stem cells and the like, or combinations thereof, with or without targeting sequences. Specific examples of therapeutic agents include, for example, pharmaceutically active compounds, proteins, cells, stem cells, oligonucleotides, ribozymes, antisense oligonucleotides, DNA compacting agents, gene/vector systems (e.g., any vehicle that allows for the uptake and expression of nucleic acids), nucleic acids (including, for example, recombinant nucleic acids; naked DNA, cDNA, RNA; genomic DNA, cDNA or RNA in a noninfectious vector or in a viral vector and which further may have attached peptide targeting sequences; antisense nucleic acid (RNA or DNA); and DNA chimeras which include gene sequences and encoding for ferry proteins such as membrane translocating sequences ("MTS") and herpes simplex virus-1 ("VP22")), and viral, liposomes and cationic and anionic polymers and neutral polymers that are selected from a number of types depending on the desired application. Non-limiting examples of virus vectors or vectors derived from viral sources include adenoviral vectors, herpes simplex vectors, papilloma vectors, adeno-associated vectors, retroviral vectors, and the like. Non-limiting examples of biologically active solutes include anti-thrombogenic agents such as heparin, heparin derivatives, urokinase, and PRACK. (dextrophenylalanine proline arginine chloromethylkctone); antioxidants such as probucol and retinoic acid; angiogenic and anti-angiogenic agents and factors; agents blocking smooth muscle cell proliferation such as rapamycin, angiopeptin, and monoclonal antibodies capable of blocking smooth muscle cell proliferation; anti-inflammatory agents such as dexamethasone, prednisolone, corticosterone, budesonide, estrogen, sulfasalazine, acetyl salicylic acid, and mesalamine; calcium entry blockers such as verapamil, diltiazem and nifedipine; antineoplastic/antiproliferative/anti-mitotic agents such as paclitaxel, 5-fluorouracil, methotrexate, doxorubicin, daunorubicin, cyclosporine, cisplatin, vinblastine, vincristine, epothilones, endostatin, angiostatin and thymidine kinase inhibitors; antimicrobials such as triclosan, dephalosporins, aminoglycosides, and nitorfurantoin; anesthetic agents such as lidocaine, buplvacaine, and ropivacaine; nitrix oxide (NO) donors such as lisidomine, molsidomine, L-argine, NO-protein adducts, NO-carbohydrate adducts, polymeric or oligomeric NO adducts; anti-coagulants such as D-Phe-Pro-Arg chloromethy! ketone, an ROD peptide- containing compound, heparine, antithrombin compounds, platelet receptor antagonists, antithrombin antibodies, anti-platelet receptor antibodies, enoxaparin, hirudin, Warafin sodium, Dicumarol, aspirin, prostaglandin inhibitors, platelet inhibitors and tick antiplatelet factors; vascular cell growth promoters such as growth factors, growth factor receptor antagonists, transcriptional activators, and translational promoters; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bi functional molecules consisting of an antibody and a cytotoxin; cholesterol-lowering agents; vasodilating agents; agents which interfere with endogenous vascoactive mechanisms; survival genes which protect against cell death, such as anti-apoptotic Bcl-2 family factors and Akt kinase; and combinations thereof

In some embodiments, stent 10 can include multiple sol-gel coatings (e.g., two sol-gel coatings, three sol-gel coatings, four sol-gel coatings, five sol-gel coatings, six sol-gel coatings, seven sol-gel coatings, eight sol-gel coatings, nine sol-gel coatings, 10 sol-gel coatings). For example, FIGS. 2A and 2B show side and cross-sectional views, respectively of a stent 100 that has three sol-gel coatings 16a, 16b, and 16c. The cross-sectional view shown in FIG. 2B is taken along the 2B-2B section line shown in FIG. 2A. Stent 100 is formed using the methods disclosed above. Typically, each of the sol-gel coatings 16a, 16b, and 16c is applied in succession. In general, each of the sol-gel coatings 16a, 16b and 16c can be prepared using similar or different sol-gel process.

Generally, sol-gel coatings 16a, 16b, and 16c can have any of the properties discussed above in connection with sol-gel coating 16. For example, each of sol-gel coatings 16a, 16b, and 16c has a thickness that can vary as discussed above. Thicknesses of each of the sol-gel coatings 16a, 16b, and 16c can be the same or different. In addition, each of sol-gel coatings 16a, 16b, and 16c can include any of the materials previously discussed. In some embodiments, some of sol-gel coatings 16a, 16b, and 16c can have the same composition. In other embodiments, the compositions of each of sol-gel coatings 16a, 16b, and 16c can be different.

In some embodiments, the combined thickness of sol-gel coatings 16a, 16b, and 16c, measured in a radial direction perpendicular to axis 18, is 1 /tm or more (e.g., 2 µm or more, 3 µm or more, 4 µm or more, 5 µm or more, 6 µm or more, 8 µm or more, 10 µm or more). In certain embodiments, the combined thickness of sol-gel coatings 16a, 16b, and 16c is 25 /tm or less (e.g., 22 µm or less, 20 µm or less, 18 µm or less, 16 µm or less, 14 µm or less, 12 µm or less). In some embodiments, the combined thickness of sol-gel coatings 16a, 16b, and 16c is between 1 µm and 25 µm (e.g., between 1 µm and 20 µm, between 1 µm and 10 µm, between 2 µm and 20 µm, between 3 µm and 18 µm, between 5 µm and 18 µm, between 5 µm and 14 µm).

The multiple sol-gel coatings on stent 100 can provide additional control over an average erosion time of stent 100 in body lumens. In addition, the compositions of each sol-gel coating 16a, 16b, and 16c can be chosen so that each coating has a particular erosion rate within body lumens. The average erosion time of stent 100 in body lumens can therefore be adjusted by adjusting the number of sol-gel coatings applied to stent 100, and/or the thicknesses of each of the applied sol-gel coatings, and/or the compositions of each of the applied sol-gel coatings.

Each of sol-gel coatings 16a. 16b, and 16c can include encapsulated therapeutic agents, pH buffers, chelating agents, and other chemical species. The encapsulated species in each of the coatings can be the same or different. In some embodiments, different chemical species can be included in each of the sol-gel coatings 16a, 16b, and 16c to achieve sequential release of the chemical species as erosion of stent 100 occurs within a body lumen. For example, a first agent can be included in sol-gel coating 16c. The first agent is released as sol-gel coating 16c erodes in a body lumen. A second agent, included within sol-gel coating 16b, is released after the first agent has been released, following erosion of sol-gel coating 16c and as sol-gel coating 16b begins to erode. After erosion of sol-gel coating 16b is complete, a third agent, included within sol-gel coating 16a, is released as sol-gel coating 16a begins to erode. In this manner, sequential release multiple chemical species within a body lumen can be achieved.

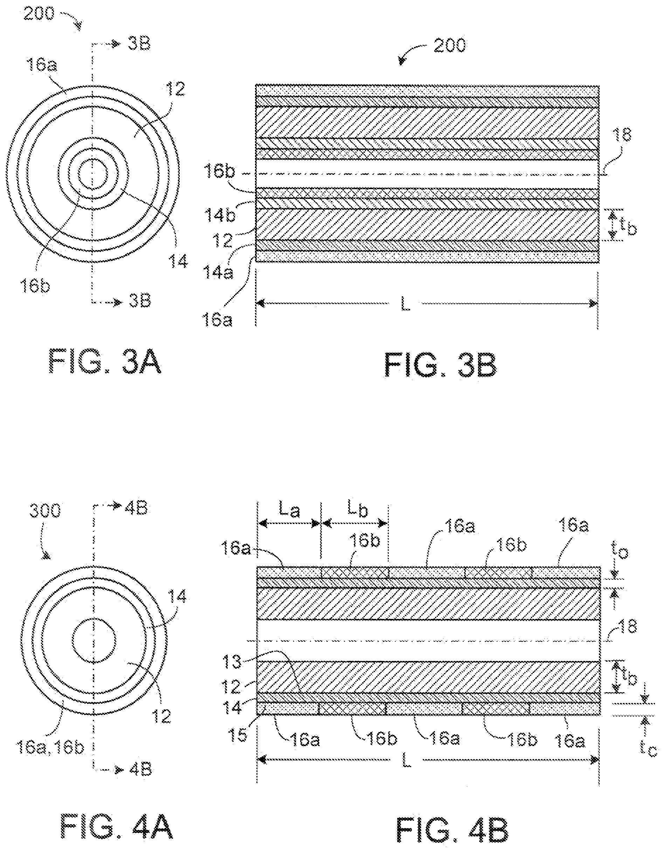

In some embodiments, sol-gel coatings can be applied to more than one surface of a stent. For example, the stent can include two different (e.g., inner and outer) intermediate layers. As an example, FIGS. 3A and 3B show side and cross-sectional views, respectively, of a stent 200 that includes intermediate layers 14a and 14b, and sol-gel coatings 16a and 16b formed on layers 14a and 14b, respectively. The cross-sectional view shown in FIG. 3B is taken along the 3B-3B section line shown in FIG. 3A. The dimensions and compositions of each of sol-gel coatings 16a and 16b can generally be similar to the dimensions and composition of sol-gel coating 16 discussed previously, for example. Each of sol-gel coatings 16a and 16b has a thickness, measured in a radial direction perpendicular to axis 18, that can generally vary as desired. In certain embodiments, the thicknesses of sol-gel coatings 16a and 16b can be the same. In other embodiments, the thicknesses of sol-gel coatings 16a and 16b can be different.

In certain embodiments, the compositions of sol-gel coatings 16a and 16b can be the same, while in other embodiments, the compositions of these coatings can be different. In general, where the properties (e.g., dimensions and/or composition) of sol-gel coatings 16a and 16b differ, the coatings can be applied in sequential fashion to stent 200. For example, portions of stent 200 where sol-gel coating 16b is to be applied can be masked using a patterned screen. Sol-gel coating 16a can then be applied to unmasked regions of stent 200. Thereafter, the patterned screen can be removed, and the portions of stent 200 where sol-gel coating 16a was applied can be masked using a second patterned screen (which can be the same or different from the patterned screen used to mask the portions of stent 200 where sol-gel coating 16b is to be applied), leaving uncoated regions of stent 200 exposed. Sol-gel coating 16b can then be applied to stent 200. Following removal of the second patterned screen, stent 200 includes portions with sol-gel coating 16a and portions with sol-gel coating 16b.

In some embodiments where the properties (e.g., dimensions and/or composition) of sol- gel coatings 16a and 16b differ, the coatings can be applied by selective methods such as high resolution spraying techniques and/or direct contact printing methods. For example, sol-gel coating 16a can be applied first to stent 200 using direct contact printing, avoiding portions of stent 200 where coating 16b is to be applied. Then, sol-gel coating 16b can be applied to stent 200, avoiding portions of stent 200 where coating 16a has been applied. As a result of this sequential coating process, stent 200 includes portions with sol-gel coating 16a and portions with sol-gel coating 16b.

In some embodiments, multiple sol-gel coating materials (e.g., two sol-gel coating materials, three sol-gel coating materials, four sol-gel coating materials, live sol-gel coating materials, six sol-gel coating materials, seven sol-gel coating materials, eight sol-gel coaling materials, nine sol-gel coating materials, 10 sol-gel coating materials) can be disposed on different regions the same intermediate layer. For example, FIGS. 4A and 4B show side and cross-sectional views, respectively, of a stent 300 that includes two sol-gel coatings 16a and 16b disposed on different regions of layer 14, The cross-sectional view shown in FIG. 4B is taken along the 4B-4B section line shown in FIG. 4A. The two sol-gel coatings have lengths Lₐ and L_{b}, respectively, measured in a direction parallel to axis IS. Each of sol-gel coatings 16a and 16b can have any of the properties discussed previously in connection with sol-gel coating 16.

Sol-gel coatings 16a and 16b can be applied to stent 300 in two steps. In a first step, portions of intermediate layer 14 where sol-gel coating 16b is to be applied can be masked (e.g., using a patterned screen) to prevent exposure of these portions to precursors of sol-gel coating 16a. Sol-gel coating 16a can then be applied to the unmasked portions of intermediate layer 14. In a second step, the patterned screen from the first step is removed and a patterned screen (different from, or the same as. the patterned screen from the first step) is positioned over portions of stent 300 where sol-gel coating 16a has been applied. Sol-gel coating 16b can then be applied to the unmasked portions of intermediate layer 14. Finally, the patterned screen covering sol-gel coating 16a can be removed. Alternatively, or in addition, sol-gel coatings 16a and 16b can be applied to portions of stent 300 using selective application methods such as direct contact printing and/or high resolution spraying.

The lengths Lₐ, and L_{b} can generally be selected as desired. In some embodiments, either or both of U, and Lb can be about 1 *µm* or more (e.g., about 5 *µm* or more, about 10 *µm* or more, about 50 *µm* or more, about 100 *µm* or more). In certain embodiments, either or both of Lₐ, and L_{b} can be about 15 mm or less (e.g., about 10 mm or less, about 5 mm or less, about 1 nun or less).

In some embodiments, the compositions of sol-gel coatings 16a and 16b are selected so that portions of stent 300 with sol-gel coating 16a erode more rapidly within body lumens than portions with sol-gel coating 16b. By selecting the composition of the sol-gel coatings, both the average erosion time of stent 300 and the erosion pattern (e.g., the shapes of fragments of stent 300 produced during erosion) can be controlled. For example, if portions of stent 300 with sol- gel coating 16a erode more rapidly than portions with sol-gel coating 16b, stent 300 will break apart during erosion into smaller tubular pieces, each having length L_{b} or less, and each of the smaller tubular pieces will further erode.

In general, many different combinations of the different types of multiple sol-gel coating materials can be used.

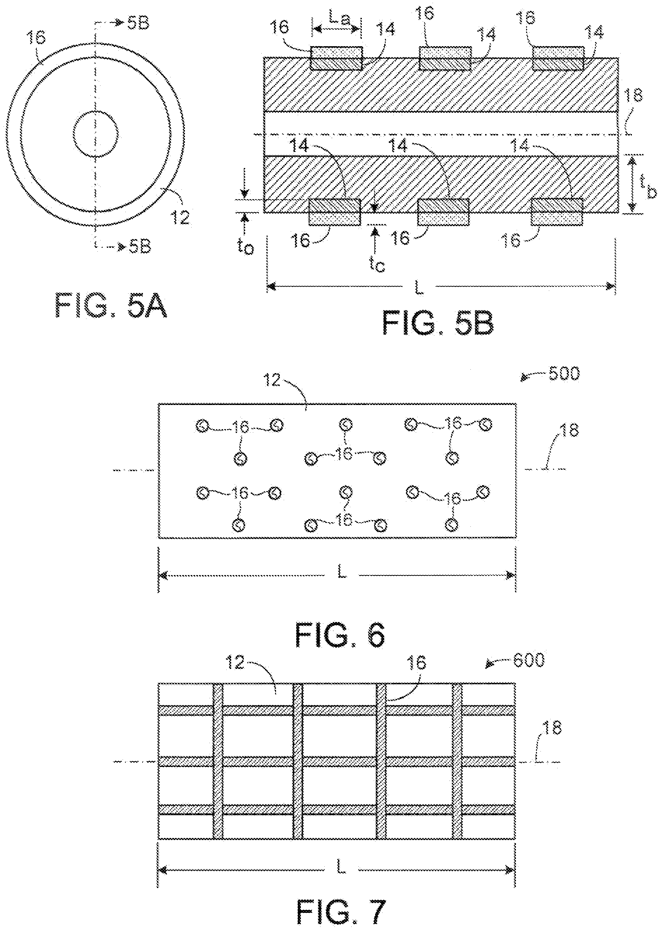

In some embodiments, sol-gel coatings can be applied only to selected portions of a stent. FIGS. 5A and 5B show side and cross-sectional views, respectively, of a stent 400 that includes a sol-gel coating 16 applied to selected portions (intermediate material regions 14) of stent 400. The cross-sectional view shown in FIG. 5B is taken along the 5B-5B section line shown in FIG. 5A. The dimensions and composition of sol-gel coating 16 can be similar to values of these parameters discussed previously in connection with other embodiments, for example. Intermediate layer 14 is also formed in selected portions of stent 400.

Stent 400 can be formed in four steps. In a first step, a masking agent is applied to selected portions of stent body 12 where sol-gel coating i6 will not be applied. In a second step, the unmasked portions of stent body 12 are anodized to produce intermediate layer 14. In a third step, sol-gel coating 16 is applied to the exposed portions of stent 400 (e.g., on intermediate layer 14 formed during the anodizing step). In a fourth step, the masking agent is removed to expose unmodified portions of stent body 12.

In certain embodiments, sol-gel coatings can be applied to a stent to form a complex pattern on a surface of the stent. As discussed above, by patterning sol-gel coatings, control over the shapes of fragments of the stent produced during erosion in a body lumen can be achieved. FIGS. 6 and 7 show two embodiments of stents with a patterned sol-gel coating 16. In FIG. 6, sol-gel coating 16 forms a series of dot-like shapes on a surface of stent 500. In FIG. 7, sol-gel coating 16 is applied so that it forms a hatched pattern on a surface of stent 600. In each of the embodiments shown in FIGS. 6 and 7, differently-shaped stent fragments according to the pattern of sol-gel coating 16 arc produced during erosion of the stents in body lumens.

Many different types of stents can be formed with sol-gel coatings. For example, stents for use in different body lumens, such as coronary stents, aortic stents, peripheral vascular stents, gastrointestinal stents, urology stents, and neurology stents, can each include sol-gel coatings. Further, any of the stents described herein can be, for example, self-expanding stents.

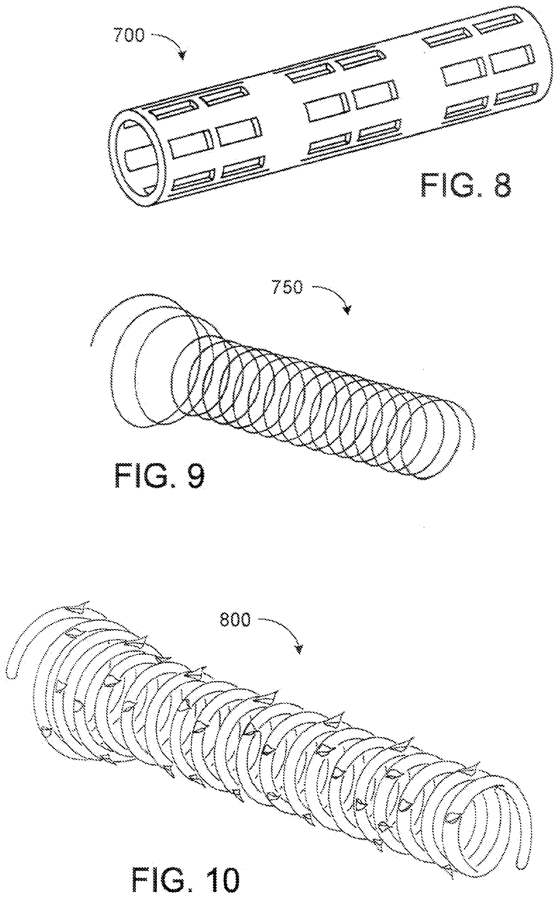

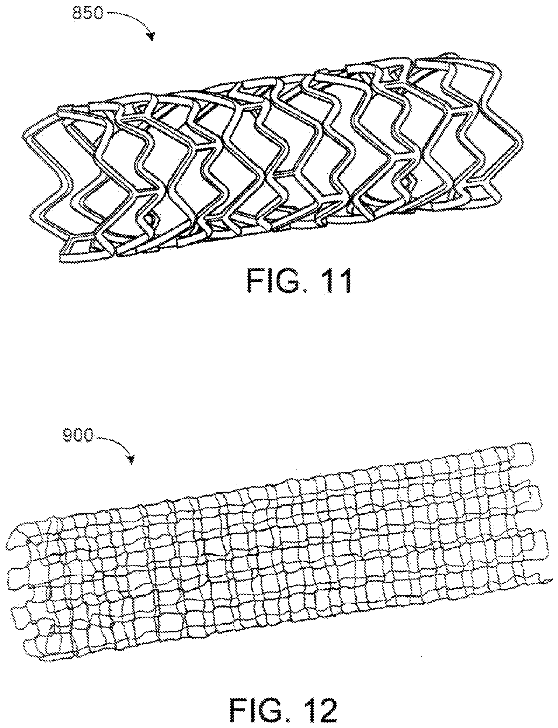

While certain embodiments have been described in which stents include a tubular stent body with a sol-gel coating, other stent shapes and geometries with sol-gel coatings can also be formed. For example, FIG. 8 shows an embodiment of a stent 700 with a tubular stent body that includes a plurality of holes extending from an outer surface of the stent to an inner surface of the stent. Sol-gel coatings can be disposed on either or both of the inner and outer surfaces of stent 700. FIGS. 9 and 10 show two embodiments of coiled stents, 750 and 800. Stents 750 and 800 can be formed from a stent material with one or more sol-gel coatings disposed thereon, and processed (e.g., by winding) into a coiled geometry. FIG. 11 shows an embodiment of a stent 850 that includes a plurality of thin portions linked in a network-like geometry. Stent 850 can be produced, for example, by forming a tubular stent with one or more sol-gel coatings as discussed previously, and then removing portions of the tubular stent (e.g., by laser cutting) to form the network-like geometry of stent 850. FIG. 12 shows an embodiment of a stent 900 that includes a stent body formed from interwoven wires. Stent 900 can be produced by forming a stent material in the shape of a wire, and forming one or more sol-gel coatings on one or more surfaces of the wire. The wire is then woven to produce stent 900. In certain embodiments, the sol-gel coated wire is interwoven to form a stent body having a chain-link type structure. In other embodiments, the sol-gel coated wire can be woven over and under other sol-gel coated wire segments to form a stent body with a mesh type structure. Each of stents 700, 750, 800, 850, and 900 can include sol-gel coatings having any of the compositions discussed previously.

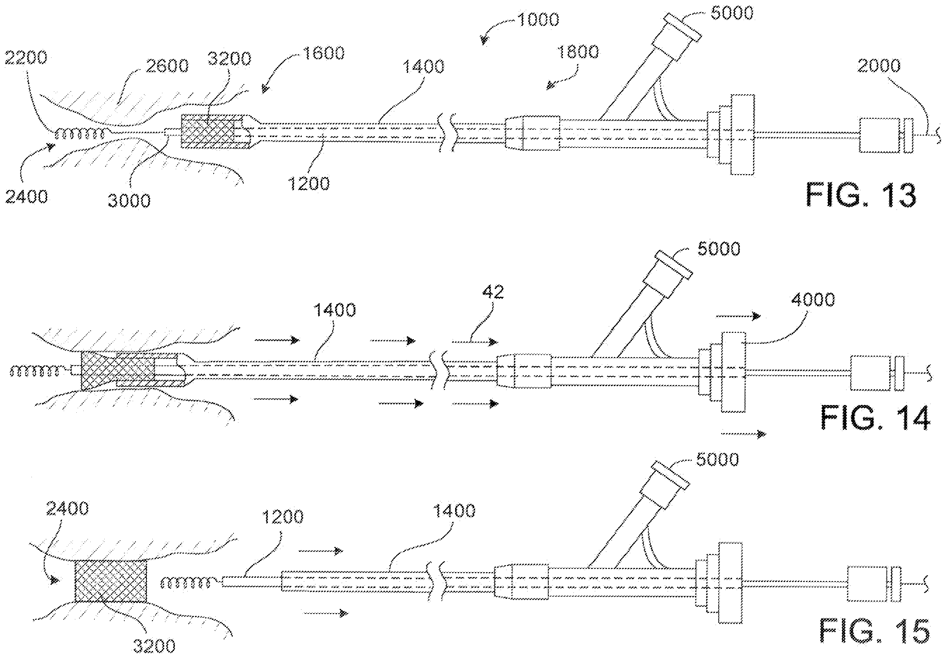

The stents described herein can be delivered, for example, into a body lumen using various techniques. As an example, FIGS. 13-15 show a system 1000 designed to deliver a self-expanding stent 3200 into a body lumen 2400 (e.g., an artery of a human). System 1000 includes a catheter 1200 and a sheath 1400 surrounding catheter 1200. Stent 3200 is positioned between catheter 1200 and sheath 1400. System 1000 includes a distal end 1600 dimensioned for insertion into body lumen 2400 and a proximal end 1800 that resides outside the body of a subject. Proximal end 1800 has at least one port 5000 and lumens for manipulation by a physician. A guide wire 2000 with a blunted end 2200 is inserted into body lumen 2400 by, for example, making an incision in the femoral artery, and directing guide wire 2000 to a constricted site 2600 of lumen 2400 (e.g., an artery constricted with plaque) using, for example, fluoroscopy as a position aid. After guide wire 2000 has reached constricted site 2600 of body lumen 2400, catheter 1200, stent 3200 and sheath 1400 are placed over the proximal end of guide wire 2000. Catheter 1200, stent 3200 and sheath 1400 are moved distally over guide wire 2000 and positioned within lumen 2400 so that stent 3200 is adjacent constricted site 2600 of lumen 2400. Sheath 1400 is moved proximally, allowing stent 3200 to expand and engage constricted site 2600. Sheath 1400, catheter 1200 and guide wire 2000 are removed from body lumen 2400, leaving stent 3200 engaged with constricted site 2600.

While certain embodiments have been disclosed, other embodiments are possible. While embodiments have been described in which the intermediate layer disposed on the stent body is made of a magnesium-containing oxide, in some embodiments, the intermediate layer disposed on the stent body can be made of different oxide materials. Generally, however, the intermediate layer disposed on the stent body is formed of one or more oxides of the material from which the stent body is formed. Examples of materials from which intermediate layer disposed on the stent body can be formed include oxides of iron and/or bismuth, oxides of noble metals such as platinum, gold, and palladium, and oxides of refractory metals such as tantalum, tungsten, molybdenum, and rhenium. In certain embodiments, where the stent body is formed of a magnesium-containing alloy material the intermediate layer can include oxides of one or more constituents of the alloy. For example, where the stent body is formed from a magnesium alloy containing iron and/or bismuth the intermediate layer can be formed from oxides of magnesium and/or iron and/or bismuth.

## Claims

1. An implantable medical endoprosthesis, comprising: an implantable medical endoprosthesis body (10) and
a sol gel coating (16) supported by the implantable medical endoprosthesis body (10), **characterized in that**
the endoprosthesis body (10) comprises magnesium, and **in that** a layer (14) comprising oxide is between the sol gel coating (16) and the implantable medical endoprosthesis body (10).

2. The implantable medical endoprosthesis of claim 1, wherein the sol gel coating (16) comprises magnesium.

3. The implantable medical endoprosthesis of claim 1, wherein the sol gel coating (16) comprises multiple layers.

4. The implantable medical endoprosthesis of claim 1, wherein the sol gel coating (16) comprises at least one therapeutic agent, optionally wherein the therapeutic agent is encapsulated.

5. The implantable medical endoprosthesis of claim 1, wherein the sol gel coating (16) comprises at least one species selected from the group consisting of proteins, chelating agents, pH buffers and antibodies.

6. The implantable medical endoprosthesis of claim 5, wherein the at least one species is encapsulated.

7. The implantable medical endoprosthesis of claim 1, wherein the oxide comprises magnesium.

8. The implantable medical endoprosthesis of claim 1, wherein the oxide is porous.

9. The implantable medical endoprosthesis of claim 1, wherein the layer (14) is supported by only some portions of stent body.

10. The implantable medical endoprosthesis of claim 1, wherein the implantable medical endoprosthesis is tube-shaped.

11. The implantable medical endoprosthesis of claim 1, wherein the implantable medical endoprosthesis is a stent, optionally wherein the endoprosthesis is a balloon-expandable stent or a self-expanding stent.

12. A delivery system for an implantable medical endoprosthesis comprising an implantable medical endoprosthesis body as described in claims 1-11.

## Patentansprüche

1. Implantierbare medizinische Endoprothese, umfassend: einen implantierbaren medizinischen Endoprothesenkörper (10) und
eine durch den implantierbaren medizinischen Endoprothesenkörper (10) gestützte Sol-Gel-Beschichtung (16), **dadurch gekennzeichnet, dass**
der Endoprothesenkörper (10) Magnesium umfasst und dass eine Oxid umfassende Schicht (14) zwischen der Sol-Gel-Beschichtung (16) und dem implantierbaren medizinischen Endoprothesenkörper (10) liegt.

2. Implantierbare medizinische Endoprothese nach Anspruch 1, wobei die Sol-Gel-Beschichtung (16) Magnesium umfasst.

3. Implantierbare medizinische Endoprothese nach Anspruch 1, wobei die Sol-Gel-Beschichtung (16) mehrere Schichten umfasst.

4. Implantierbare medizinische Endoprothese nach Anspruch 1, wobei die Sol-Gel-Beschichtung (16) mindestens ein Therapeutikum umfasst, wahlweise wobei das Therapeutikum eingekapselt ist.

5. Implantierbare medizinische Endoprothese nach Anspruch 1, wobei die Sol-Gel-Beschichtung (16) mindestens eine Spezies umfasst, die aus der aus Proteinen, Komplexbildnern, pH-Puffern und Antikörpern bestehenden Gruppe ausgewählt ist.

6. Implantierbare medizinische Endoprothese nach Anspruch 5, wobei die mindestens eine Spezies eingekapselt ist.

7. Implantierbare medizinische Endoprothese nach Anspruch 1, wobei das Oxid Magnesium umfasst.

8. Implantierbare medizinische Endoprothese nach Anspruch 1, wobei das Oxid porös ist.

9. Implantierbare medizinische Endoprothese nach Anspruch 1, wobei die Schicht (14) von lediglich einigen Abschnitten des Stentkörpers gestützt ist.

10. Implantierbare medizinische Endoprothese nach Anspruch 1, wobei die implantierbare medizinische Endoprothese röhrenförmig ist.

11. Implantierbare medizinische Endoprothese nach Anspruch 1, wobei die implantierbare medizinische Endoprothese ein Stent ist, wahlweise wobei die Endoprothese ein ballon-expandierbarer Stent oder ein selbstexpandierender Stent ist.

12. Zuführsystem für eine implantierbare medizinische Endoprothese, umfassend einen implantierbaren medizinischen Endoprothesenkörper nach Ansprüchen 1-11.

## Revendications

1. Endoprothèse médicale implantable comprenant :
un corps d'endoprothèse implantable (10) et
un revêtement sol-gel (16) supporté par le corps d'endoprothèse médicale implantable (10), **caractérisée en ce que**
le corps d'endoprothèse (10) comprend du magnésium, et **en ce qu'**une couche (14) comprenant un oxyde est située entre le revêtement sol-gel (16) et le corps d'endoprothèse médicale implantable (10).

2. Endoprothèse médicale implantable selon la revendication 1, dans laquelle le revêtement sol-gel (16) comprend du magnésium.

3. Endoprothèse médicale implantable selon la revendication 1, dans laquelle le revêtement sol-gel (16) comprend de multiples couches.

4. Endoprothèse médicale implantable selon la revendication 1, dans laquelle le revêtement sol-gel (16) comprend au moins un agent thérapeutique, éventuellement dans laquelle l'agent thérapeutique est encapsulé.

5. Endoprothèse médicale implantable selon la revendication 1, dans laquelle le revêtement sol-gel (16) comprend au moins une espèce sélectionnée dans le groupe constitué de protéines, d'agent chélateurs, de tampons de pH et d'anticorps.

6. Endoprothèse médicale implantable selon la revendication 5, dans laquelle la ou les espèces sont encapsulées.

7. Endoprothèse médicale implantable selon la revendication 1, dans laquelle l'oxyde comprend du magnésium.

8. Endoprothèse médicale implantable selon la revendication 1, dans laquelle l'oxyde est poreux.

9. Endoprothèse médicale implantable selon la revendication 1, dans laquelle la couche (14) est supportée par seulement certaines parties de corps de stent.

10. Endoprothèse médicale implantable selon la revendication 1, l'endoprothèse médicale implantable étant de forme tubulaire.

11. Endoprothèse médicale implantable selon la revendication 1, l'endoprothèse médicale implantable étant un stent, éventuellement l'endoprothèse étant un stent expansible par ballonnet ou un stent auto-expansible.

12. Système de pose pour une endoprothèse médicale implantable comprenant un corps d'endoprothèse médicale implantable tel que décrit dans les revendications 1 à 11.
